# EUROPÄISCHE PATENTSCHRIFT

(11) **EP 2 148 626 B1**
(45) Veröffentlichungstag und Bekanntmachung des Hinweises auf die Patenterteilung: **04.11.2015**
(21) Anmeldenummer: 08758724.2
(22) Anmeldetag: 23.05.2008
(51) Int. Cl.: A61B 17/88, A61B 17/68, A61B 17/82

(54) **Chirurgisches Instrument zum Gegeneinanderspannen von plattenförmigen Anlageelementen**
Surgical instrument for tensioning plate-shaped bearing elements relative to each other
Instrument chirurgical destiné à serrer des éléments d'appui en forme de plaque les uns contre les autres

(30) Priorität: 25.05.2007 DE 102007026079
(43) Veröffentlichungstag der Anmeldung: 03.02.2010
(73) Patentinhaber: Aesculap AG, 78532 Tuttlingen (DE)
(72) Erfinder: LUTZE, Theodor, 78582 Balgheim (DE); DWORSCHAK, Manfred, 78589 Dürbheim (DE); MORALES, Pedro, 78532 Tuttlingen (DE); WEISSHAUPT, Dieter, 78194 Immendingen (DE)
(74) Vertreter: Hoeger, Stellrecht & Partner Patentanwälte mbB
(86) Internationale Anmeldenummer: PCT/EP2008/004129
(87) Internationale Veröffentlichungsnummer: WO 2008/145307

(56) Entgegenhaltungen:
- DE-B3- 10 310 004
- DE-U1-202006 007 221

## Beschreibung

Die Erfindung betrifft ein chirurgisches Instrument zum Gegeneinanderspannen von zwei plattenförmigen Anlageelementen an gegenüberliegenden Seiten von Knochenstücken, wobei ein erstes Anlageelement im Abstand nebeneinander zwei durch Öffnungen des zweiten Anlageelementes hindurchgreifende stift- oder stabförmige Verbindungsglieder trägt, mit einer Abstützfläche zur Anlage an der dem ersten Anlageelement abgewandten Seite des zweiten Anlageelementes und mit einer an den Verbindungsgliedern angreifenden Spanneinrichtung zur Verschiebung der Verbindungsglieder und damit des ersten Anlageelementes in Richtung auf das zweite Anlageelement.

Ein derartiges Instrument ist bekannt aus der DE 197 00 474 und auch aus der DE 103 10 004 B3, die jedoch beide nur zum Ergreifen eines einzigen stiftförmigen Verbindungselementes ausgebildet sind. In der DE 20 2006 007 221 U1 ist ein Instrument beschrieben, bei dem die Anlageelemente auch bei einem Implantat gegeneinander gespannt werden können, das nebeneinander zwei stiftförmige Verbindungselemente aufweist. Dabei greift das Instrument zum Spannen an einer Brücke an, die die beiden stiftförmigen Verbindungselemente an ihren freien Enden starr miteinander verbindet. Dadurch werden die beiden plattenförmigen Anlageelemente zwangsläufig parallel zueinander verschoben, wenn die Spanneinrichtung betätigt wird, so dass die Anlageelemente einer nicht exakt parallelen Struktur der zwischen ihnen aufgenommenen Knochenplatten nicht folgen können. Dies führt zu unterschiedlichen Werten des Anpressdruckes der Anlageelemente an den dazwischenliegenden Knochenstücken und damit zu unterschiedlichen Spannungen. Eine derartig ungleichmäßige Spannungsverteilung kann ungünstig sein.

Es ist Aufgabe der Erfindung, bei einem gattungsgemäßen Instrument zum Gegeneinanderspannen von Anlageelementen mit zwei nebeneinander angeordneten Verbindungselementen eine Anpassung der Anlageelemente an unterschiedliche Formen und Dicken der zwischen den Anlageelementen liegenden Knochenstücke zu ermöglichen mit dem Ergebnis einer möglichst gleichmäßigen Verteilung der Anpresskräfte über die gesamte Fläche der Anlageelemente.

Diese Aufgabe wird bei einem chirurgischen Instrument der eingangs beschriebenen Art erfindungsgemäß dadurch gelöst, dass das chirurgische Instrument nebeneinander zwei lösbar mit je einem Verbindungsglied verbindbare Greifelemente umfasst, und dass die Spanneinrichtung über ein Ausgleichselement an den Greifelementen angreift, welches beim Spannen der Spanneinrichtung eine unterschiedlich große Verschiebung der beiden Greifelemente und dadurch der beiden Verbindungsglieder zulässt.

Die Spanneinrichtung greift also nicht unmittelbar an den Greifelementen an, sondern über ein Ausgleichselement, welches die Verschiebebewegung der Spanneinrichtung so auf die beiden Greifelemente überträgt, dass diese Greifelemente unterschiedliche Verschiebebewegungen ausführen können. Dies führt dazu, dass die Anlageelemente sich an die Kontur der dazwischenliegenden Knochenstücke anpassen können, sie werden nicht zwangsläufig parallel zueinander verschoben, sondern sie können sich auch in geringem Umfange gegeneinander verschwenken und neigen. Dadurch wird erreicht, dass die Anpresskräfte im gesamten Bereich der plattenförmigen Anlageelemente gleichmäßig auf die Knochenstücke verteilt werden, es treten keine Spannungsspitzen mehr auf.

Gemäß einer bevorzugten Ausführungsform kann vorgesehen sein, dass das Ausgleichselement als Tragarm ausgebildet ist, an dem im Abstand zueinander die Greifelemente gehalten sind, und an dem zwischen den Haltepunkten der Greifelemente die Spanneinrichtung derart angreift, dass der Arm relativ zur Spannrichtung der Spanneinrichtung verschwenkbar ist.

Insbesondere kann vorgesehen sein, dass die Greifelemente in ihrem Haltepunkt derart an dem Tragarm gehalten sind, dass dieser gegenüber den Greifelementen verschwenkbar ist. Der Tragarm hat ähnlich wie ein Waagebalken somit die Möglichkeit, die Verschiebebewegung, die ihm durch die Spanneinrichtung vermittelt wird, in ungleichem Maße auf die beiden Greifelemente zu übertragen, wenn der Tragarm verschwenkt wird, werden die Greifelemente auf einer Seite stärker verschoben als der Tragarm selbst, auf der gegenüberliegenden Seite dagegen weniger.

Die nachfolgende Beschreibung bevorzugter Ausführungsformen der Erfindung dient im Zusammenhang mit der Zeichnung der näheren Erläuterung. Es zeigen:

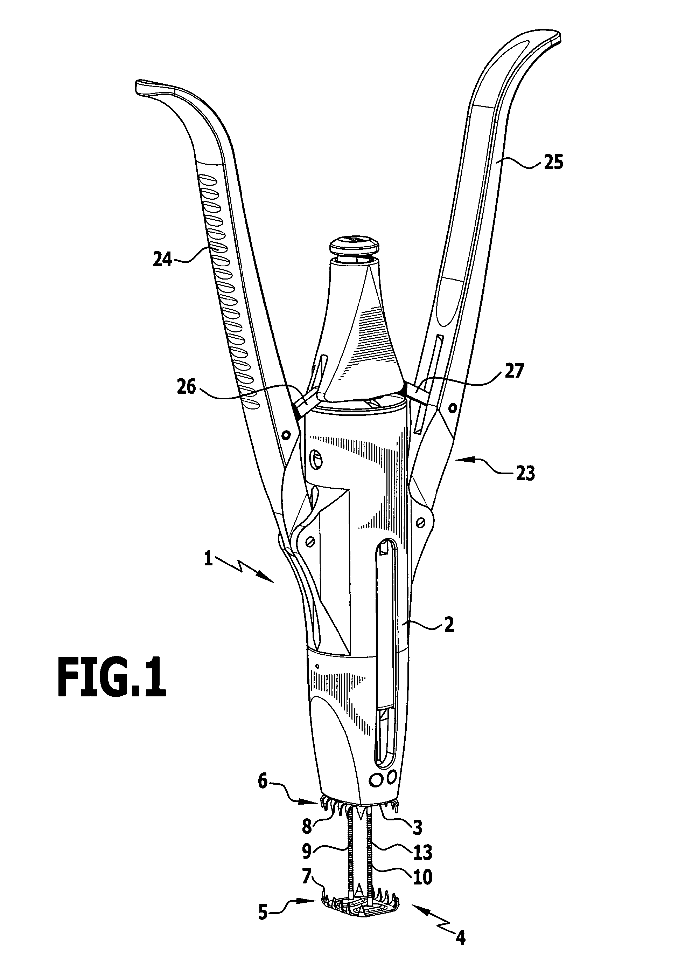

- Figur 1:: eine perspektivische Ansicht eines chirurgischen Instrumentes zum Gegeneinanderspannen von zwei plattenförmigen Anlageelementen;

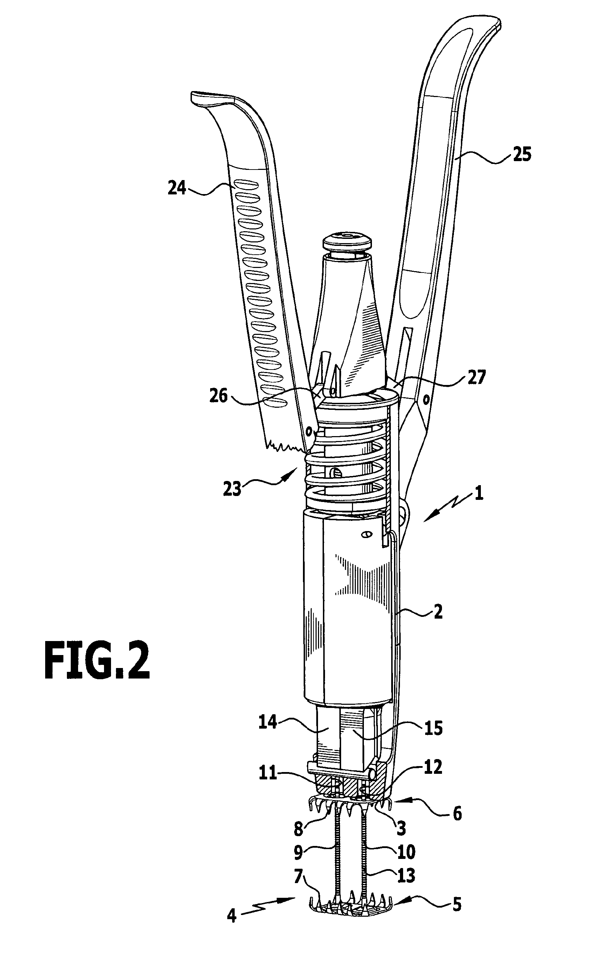

- Figur 2:: eine Ansicht ähnlich Figur 1 mit aufgeschnittenem Gehäuse;

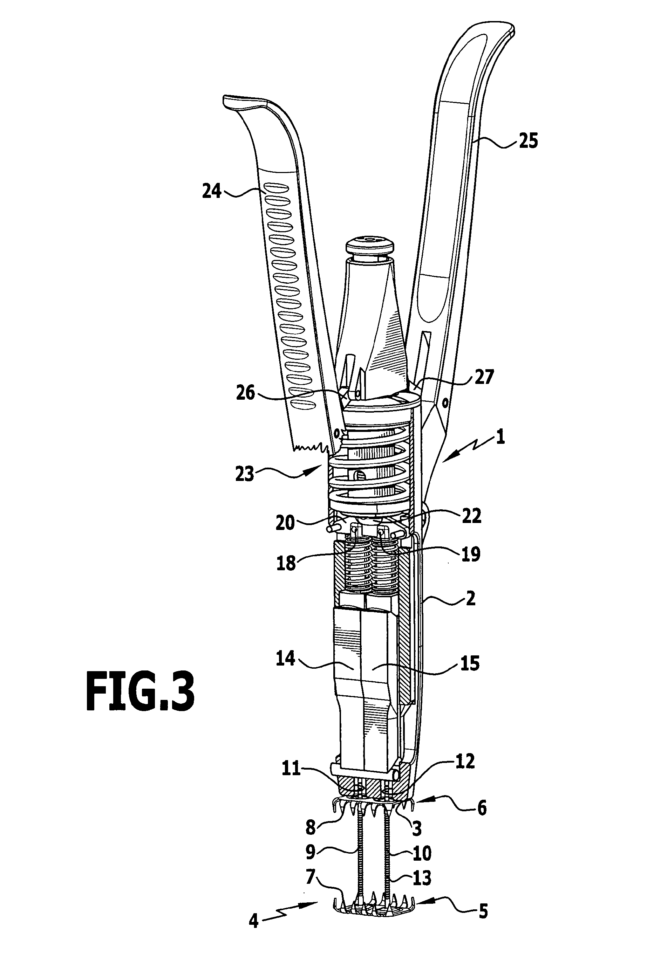

- Figur 3:: eine Ansicht ähnlich Figur 2 nach Entfernung einer Spannhülse für die Greifelemente;
- Figur 4:: eine Längsschnittansicht durch das Instrument der Figuren 1 bis 3 mit parallel zueinanderstehenden plattenförmigen Anlageelementen und
- Figur 5:: eine Ansicht ähnlich Figur 4 mit geringfügig gegeneinander verschwenkten plattenförmigen Anlageelementen.

Das in der Zeichnung dargestellte Instrument 1 ist sehr ähnlich aufgebaut wie das Instrument, das in der DE 103 10 004 B3 beschrieben ist. Auf die entsprechende Beschreibung dieser vorveröffentlichten Druckschrift wird daher ausdrücklich Bezug genommen.

Dieses Instrument 1 weist ein im Wesentlichen zylindrisches Gehäuse 2 auf, an dessen unterem, distalem Ende eine Anlagefläche 3 ausgebildet ist, mit der das Instrument 1 an ein Implantat 4 angelegt werden kann.

Dieses Implantat umfasst eine erste, untere Anlageplatte 5 sowie eine zweite, obere Anlageplatte 6, die beide mit am Rand angeordnetem, aus der Ebene der Anlageplatten hervorstehenden und in Richtung auf die jeweils andere Anlageplatte weisenden spitzen Zähnen 7, 8 versehen sind. Die erste Anlageplatte trägt parallel nebeneinander verlaufend zwei stiftförmige, längere Verbindungselemente 9, 10, die durch entsprechende Öffnungen in der zweiten Anlageplatte 6 hindurchgreifen und durch Öffnungen 11, 12 in der Anlagefläche 3 des Gehäuses 2 in das Innere des Instrumentes 1 ragen.

Die zweite Anlageplatte 6 kann längs der Verbindungselemente 9, 10 gegen die erste Anlageplatte 5 verschoben werden und greift dabei in aus der Zeichnung nicht ersichtlicher Weise rastend in Umfangsnuten 13 der Verbindungselemente 9, 10 ein, so dass die beiden Anlageplatten zwar einander angenähert werden können, aber nicht mehr voneinander entfernt werden können.

Im Inneren des Gehäuses 2 sind zwei gleich aufgebaute Greifelemente 14, 15 nebeneinander angeordnet, jedes der beiden Greifelemente 14, 15 ist einem der beiden Verbindungselemente 9, 10 eines Implantates 4 zugeordnet. Die genaue Ausgestaltung der Greifelemente ist im Zusammenhang mit der vorliegenden Erfindung von untergeordneter Bedeutung, hier wird auf die Konstruktion gemäß der DE 103 10 004 verwiesen, die der vorliegenden Konstruktion weitgehend entspricht.

Wesentlich ist, dass beide Greifelemente 14, 15 jeweils eines der beiden Verbindungselemente 9, 10 im Bereich einer Verdickung 16, 17 umgreifen können, so dass eine feste Zugvermittlung zwischen den Greifelementen 14, 15 und den Verbindungselementen 9, 10 entsteht, das heißt beim Verschieben der Greifelemente 14, 15 längs der Verbindungselemente 9, 10 können Zugkräfte auf die Verbindungselemente 9, 10 ausgeübt werden.

Die beiden Greifelemente 14, 15 sind jeweils um parallel zueinander und senkrecht zur Längsachse der Verbindungselemente 9, 10 verlaufende Schwenkachsen 18, 19 verschwenkbar mit einem im oberen Teil des Gehäuses 2 angeordneten und das Gehäuse quer durchsetzenden Tragarm 20 verbunden, der in der Mitte zwischen den Anlenkpunkten der beiden Greifelemente 14, 15 seinerseits um eine parallel zu den Schwenkachsen 18, 19 verlaufende Schwenkachse 21 verschwenkbar mit einem Zugglied 22 einer Spanneinrichtung 23 verbunden ist. Dieses Zugglied 22 kann in proximale Richtung verschoben werden und zieht dadurch die Verbindungselemente 9, 10 in das Innere des Gehäuses 2 hinein.

Zur Betätigung der Spanneinrichtung 23 sind am Gehäuse an dessen Außenseite zwei einander gegenüberliegende Schwenkhebel 24, 25 verschwenkbar gelagert, die über Kniehebel 26, 27 das Zugglied 22 verschieben können. Diese Verbindung erfolgt unter Zwischenschaltung eines Tellerfedernpaketes 28, das bei geringen Spannkräften unverformt bleibt und erst komprimiert wird, wenn die von den Schwenkhebeln 24, 25 erzeugten Zugkräfte einen bestimmten Schwellwert überschreiten. Dadurch wirkt das Tellerfedernpaket 28 als Kraftbegrenzer. Diese Konstruktion entspricht weitgehend der Konstruktion der DE 103 10 004 B3 und darauf wird ausdrücklich Bezug genommen.

Für die vorliegende Erfindung von Bedeutung ist die Zwischenschaltung des Tragarmes 20 zwischen das Zugglied 22 einerseits und die Greifelemente 14, 15 andererseits. Durch diesen verschwenkbaren Tragarm wird nämlich ermöglicht, dass der Verschiebeweg, den das Zugglied 22 bei der Betätigung der Spanneinrichtung 23 erfährt, in unterschiedlichem Maße auf die beiden Greifelemente 14, 15 übertragen wird.

Wenn der Tragarm unverschwenkt bleibt, werden beide Greifelemente 14, 15 in gleicher Weise verschoben, der Verschiebeweg ist dann gleich groß wie der Verschiebeweg des Zuggliedes 22. Wenn jedoch bei dieser Verschiebung der Tragarm verschwenkt wird, ist der Verschiebeweg der Greifelemente 14, 15 unterschiedlich groß, auf der abgesenkten Seite des Tragarmes ist der Verschiebeweg kleiner als der des Zuggliedes, auf der angehobenen Seite des Tragarmes dagegen größer.

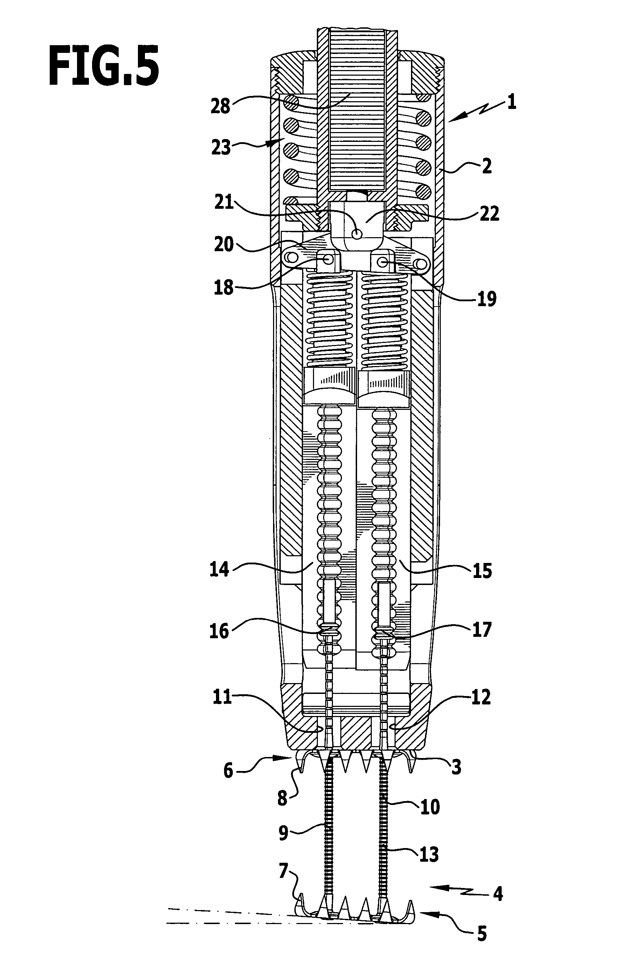

Dies führt dazu, dass auch die erste Anlageplatte 5 an einer Seite stärker der zweiten Anlageplatte angenähert wird als an der gegenüberliegenden Seite, wie dies in Figur 5 dargestellt ist, das heißt die beiden Anlageplatten werden beim Annähern geringfügig gegeneinander geneigt, so dass sie sich optimal an die Kontur der dazwischenliegenden, in der Zeichnung nicht dargestellten Knochenstücke anpassen können.

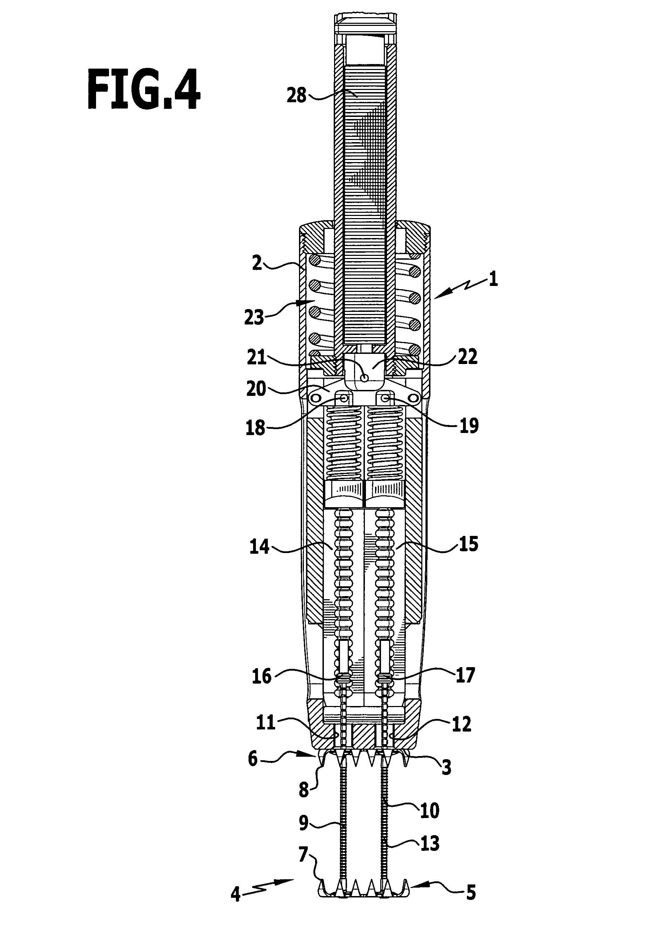

Wenn der Tragarm selbst nicht verschwenkt wird, werden die Anlageplatten dagegen beim Spannen parallel zueinander verschoben, wie dies in Figur 4 dargestellt ist.

Durch das Vorsehen des verschwenkbaren Tragarmes, der eine verschwenkbare Ausgleichsbrücke zwischen der Spanneinrichtung und den beiden Greifelementen bildet, ist somit eine an die Dicke der zwischen den Anlageplatten angeordneten Knochenstücke angepasste Verschiebung der Anlageplatten möglich, so dass die Zähne der Anlageplatten längs des gesamten Umfanges der Anlageplatten im wesentlichen mit einer gleich großen Kraft gegen die Knochenstücke gedrückt werden, Spannungsspitzen können dadurch vermieden werden.

Durch die Kraftbegrenzung, die in Form des Tellerfedernpaketes 28 vorgesehen ist, wird auch dafür Sorge getragen, dass die Maximalkräfte einen bestimmten Wert nicht überschreiten, und auch dies trägt im Zusammenhang mit der Verschwenkbarkeit des Tragarmes dazu bei, dass beide Anlageplatten längs ihrer gesamten Fläche gleichmäßig gegeneinander gespannt werden können.

## Patentansprüche

1. Chirurgisches Instrument (1) zum Gegeneinanderspannen von zwei plattenförmigen Anlageelementen an gegenüberliegenden Seiten von Knochenstücken, wobei ein erstes Anlageelement im Abstand nebeneinander zwei durch Öffnungen des zweiten Anlageelementes hindurchgreifende stift- oder stabförmige Verbindungsglieder (9, 10) trägt, mit einer Abstützfläche zur Anlage an der dem ersten Anlageelement abgewandten Seite des zweiten Anlageelementes und mit einer an den Verbindungsgliedern (9, 10) angreifenden Spanneinrichtung (23) zur Verschiebung der Verbindungsglieder (9, 10) und damit des ersten Anlageelementes in Richtung auf das zweite Anlageelement, **dadurch gekennzeichnet, dass** das chirurgische Instrument (1) nebeneinander zwei lösbar mit je einem Verbindungsglied (9, 10) verbindbare Greifelemente (14, 15) umfasst und dass die Spanneinrichtung (23) über ein Ausgleichselement (20) an den Greifelementen (14, 15) angreift, welches beim Spannen der Spanneinrichtung (23) eine unterschiedlich große Verschiebung der beiden Greifelemente (14, 15) und dadurch der beiden Verbindungsglieder (9,10) zulässt.

2. Chirurgisches Instrument nach Anspruch 1, **dadurch gekennzeichnet, dass** das Ausgleichselement als Tragarm (20) ausgebildet ist, an dem im Abstand zueinander die Greifelemente (14, 15) gehalten sind und an dem zwischen den Haltepunkten der Greifelemente (14, 15) die Spanneinrichtung (23) derart angreift, dass der Tragarm (20) relativ zur Spannrichtung der Spanneinrichtung (23) verschwenkbar ist.

3. Chirurgisches Instrument nach Anspruch 2, **dadurch gekennzeichnet, dass** die Greifelemente (14, 15) in ihrem Haltepunkt derart an dem Tragarm (20) gehalten sind, dass dieser gegenüber den Greifelementen (14, 15) verschwenkbar ist.

## Claims

1. Surgical instrument (1) for tensioning two plate-shaped engaging elements with respect to each other at opposite sides of bone portions, a first engaging element carrying next to each other at a spacing from each other two pin-shaped or rod-shaped connecting members (9, 10) which pass through openings (11, 12) in the second engaging element, with a supporting surface for engagement on the side of the second engaging element that faces away from the first engaging element, and with a tensioning device (23) engaging the connecting members (9, 10) for displacement of the connecting members (9, 10) and consequently of the first engaging element in the direction towards the second engaging element, **characterized in that** the surgical instrument (1) comprises next to each other two grip elements (14, 15), each releasably connectable to a connecting member (9, 10), and **in that** the tensioning device (23) engages the grip elements (14, 15) through a compensating element (20) which, upon tensioning of the tensioning device (23), allows a differently sized displacement of the two grip elements (14, 15) and consequently of the two connecting members (9, 10).

2. Surgical instrument in accordance with claim 1, **characterized in that** the compensating element is configured as a carrying arm (20), on which the grip elements (14, 15) are held at a spacing from each other, and on which the tensioning device (23) engages between the holding points of the grip elements (14, 15) in such a way that the carrying arm (20) is pivotable relative to the tensioning direction of the tensioning device (23).

3. Surgical instrument in accordance with claim 2, **characterized in that** the grip elements (14, 15) are held at their holding point on the carrying arm (20) in such a way that the carrying arm is pivotable relative to the grip elements (14, 15).

## Revendications

1. Instrument chirurgical (1) pour serrer l'un contre l'autre deux éléments d'appui en forme de plaque sur des côtés opposés de parties d'os, instrument dans lequel un premier élément d'appui porte côte à côte, à distance l'un de l'autre, deux organes de liaison (9, 10) en forme de broche ou de barre, qui s'engagent à travers des ouvertures du deuxième élément d'appui, avec une surface d'application destinée à venir s'appuyer contre le côté du deuxième élément d'appui, qui est opposé à celui dirigé vers le premier élément d'appui, et avec un dispositif de serrage (23) agissant sur les organes de liaison (9, 10) pour faire coulisser les organes de liaison (9, 10) et ainsi le premier élément d'appui en direction du deuxième élément d'appui,
**caractérisé en ce que** l'instrument chirurgical (1) comprend côte à côte, deux éléments de pince (14, 15) pouvant être reliés chacun de manière amovible à un organe de liaison (9, 10), et **en ce que** le dispositif de serrage (23) agit sur les éléments de pince (14, 15) par l'intermédiaire d'un élément de compensation (20), qui, lors du serrage du dispositif de serrage (23), permet un coulissement de grandeur différente des deux éléments de pince (14, 15) et ainsi des deux organes de liaison (9, 10).

2. Instrument chirurgical selon la revendication 1,
**caractérisé en ce que** l'élément de compensation est réalisé sous la forme d'un bras porteur (20) sur lequel sont maintenus, à distance l'un de l'autre, les éléments de pince (14, 15), et sur lequel agit, entre les points de maintien des éléments de pince (14, 15), le dispositif de serrage (23), de façon telle que le bras porteur (20) puisse pivoter par rapport à la direction de serrage du dispositif de serrage (23).

3. Instrument chirurgical selon la revendication 2,
**caractérisé en ce que** les éléments de pince (14, 15) sont maintenus en leur point de maintien sur le bras porteur (20) de manière à ce que celui-ci puisse pivoter par rapport aux éléments de pince (14, 15).
